# EUROPÄISCHE PATENTANMELDUNG

(11) **EP 1 627 684 A1**
(43) Veröffentlichungstag der Anmeldung: **22.02.2006**
(21) Anmeldenummer: 04019759.2
(22) Anmeldetag: 20.08.2004
(51) Int. Cl.: B01L 3/00, A61B 5/00, A61B 5/15

(54) **Mikrofluidiksystem und Verfahren zu dessen Herstellung**

(71) Anmelder: F. HOFFMANN-LA ROCHE AG, 4070 Basel (CH); Roche Diagnostics GmbH, 68305 Mannheim (DE)
(72) Erfinder: Sarofim, Emad, 6300 Zug (CH); Calasso, Irio, Dr., 6415 Arth (CH); Griss, Patrick, Dr., 8004 Zürich (CH)
(74) Vertreter: Pfiz, Thomas

(57) **Zusammenfassung**

Die Erfindung betrifft ein Mikrofluidiksystem mit einem mit einem Stechorgan (14) versehenen Trägerkörper (12) und einem darauf befindlichen halboffenen Mikrokanal (16) zum kapillaren Transport einer Probenflüssigkeit von einer Aufnahme- zu einer Zielstelle (22,24). Um ein höheres Aspektverhältnis zu erhalten, wird vorgeschlagen, dass der Trägerkörper (12) mit einer den Mikrokanal (16) zumindest im oberen Bereich seitlich begrenzenden Aufbauschicht (18) für den Flüssigkeitstransport beschichtet ist
(Fig. 1)

## Beschreibung

Die Erfindung betrifft ein Mikrofluidiksystem mit einem vorzugsweise mit einem Stechorgan versehenen Trägerkörper und einem darauf befindlichen halboffenen Mikrokanal zum kapillaren Transport einer Flüssigkeit von einer Aufnahme- zu einer Zielstelle. Die Erfindung betrifft weiter eine bevorzugte Verwendung eines solchen Systems sowie ein Verfahren zu dessen Herstellung.

Systeme dieser Art erlauben speziell in der Bioanalytik die Untersuchung geringster Fluidmengen, wie sie beispielsweise für Blutglucosebestimmungen in situ als Kapillarblut entnommen werden. Die Mikrofluidik zeichnet sich dabei neben den mikroskopischen Volumina (Mikroliter und weniger) auch durch immer kleiner dimensionierte Strukturelemente aus, die eine Ausnutzung von Kapillarkräften erlauben und in so genannten Disposibles kostengünstig und für eine Massenfertigung geeignet realisiert werden müssen. Aus dem Bereich der Halbleitertechnologie sind zwar solche Verfahren speziell in Form des Maskenätzens ("photochemical etching") für hochintegrierte Systeme bekannt, die dort verwendeten Materialien lassen sich aber vor allem aufgrund ihrer Sprödigkeit für mechanisch beanspruchte Strukturen kaum einsetzen. Beim Ätzen von biokompatiblen Materialien wie Stahl tritt aufgrund des isotropen Materialabtrags das Problem auf, dass die Querschnitte der erzeugten Kanalstrukturen keinen besonders optimalen Flüssigkeitstransport erlauben. Die in diesem Zusammenhang in der US-A 2002/0168290 bereits vorgeschlagene Applikation von Netzmitteln ist aus folgenden Gründen problematisch:
- Es ist ein zusätzlicher Herstellungsschritt erforderlich.
- Typischerweise wird Kompatibilität zu einem Nachweisverfahren eines Analyten in der transportierten Probe gefordert (d. h. keine Beeinflussung bzw. inakzeptable Verfälschung des Messresultats).
- In der Regel muss sogar eine Bio-Kompatibilität (keinerlei toxische Effekte) gegeben sein, da bei der Probenentnahme nicht ausgeschlossen werden kann, dass mit dem Netzmittel beschichtet Teile kurzzeitig in den Organismus gelangen.
- Die Hydrophilisierung muss ausreichend lagerstabil sein.
- Es bestehen physikalische Beschränkungen bei der alleinigen Anwendung eines Netzmittels ohne geeignete Geometrie. Solche Limitierungen liegen einzeln oder in Kombination in der geforderten Transportweite, Lage/Gravitationsunabhängigkeit und/oder Fließgeschwindigkeit.

Ausgehend hiervon liegt der Erfindung die Aufgabe zugrunde, die im Stand der Technik aufgetretenen Nachteile zu vermeiden und ein System und ein Herstellungsverfahren dahingehend zu verbessern, dass Strukturen für einen effektiven Transport von geringen Flüssigkeitsmengen mit günstigen Maßnahmen geschaffen werden. Insbesondere sollen allfällige Limitierungen, die auf der alleinigen Anwendung von Netzmitteln beruhen, reduziert werden.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Dementsprechend wird im Hinblick auf ein Mikrofluidiksystem vorgeschlagen, dass der Trägerkörper mit einer den Mikrokanal zumindest im oberen Bereich seitlich begrenzenden Aufbauschicht für den Flüssigkeitstransport beschichtet ist. Die Beschichtung erlaubt auf einfache Weise eine fest haftende Strukturbildung mit einer zuvor formlosen Substanz, wobei durch die Kanalbildung oder Kanalerhöhung in der Aufbauschicht eine Flüssigkeit führende fluidische Funktion realisiert wird, die auf einer Erhöhung der Kapillarität beruht. Speziell bedeutet dies, dass auch auf isotrop ätzbaren Substraten Kanalquerschnitte mit hohem Aspektverhältnis realisiert werden können, welche die Kapillarwirkung entscheidend verbessern.

In vorteilhafter Ausgestaltung weist der Mikrokanal einen in den Trägerkörper eingebrachten, vorzugsweise eingeätzten unteren Querschnittsbereich und einen darüber liegenden, in der Aufbauschicht ausgebildeten oberen Querschnittsbereich auf. Möglich ist es auch, dass die Aufbauschicht den Mikrokanal über seine gesamte Tiefe seitlich begrenzt und somit allein eine flüssigkeitsführende Funktion übernimmt.

Besonders bevorzugt ist es, wenn die Aufbauschicht aus einem Fotolack, vorzugsweise einem Dickfilm-Fotolack besteht. Damit lassen sich auf einfache Weise mikrofluidische Strukturen auf einem Träger, welcher die erforderliche Festigkeit und Inertheit für den Einsatzzweck besitzt, realisieren. Dies kann dadurch erfolgen, dass die Aufbauschicht zur Bildung oder Erhöhung des Mikrokanals fotostrukturiert ist, so dass auch komplexe Geometrien mit der erforderlichen Genauigkeit geschaffen werden.

Vorteilhafterweise besitzt die Aufbauschicht eine Schichtdicke von mehr als 50 µm, vorzugsweise 200 bis 500 µm.

Ein weiterer Erfindungsaspekt besteht darin, dass der Mikrokanal mehrere durch sukzessive Ätzschritte von einer Oberfläche des Trägerkörpers her in die Tiefe eingeätzte Teilquerschnitte aufweist. Auch auf diese Weise ist es in einem isotrop ätzbaren Trägermaterial möglich, ein großes Verhältnis aus Tiefe zu Breite des Mikrokanals zu erzielen. Besonders günstig ist es, wenn dieses Aspektverhältnis größer als 0,5, vorzugsweise größer als 0,8 ist.

Für einen selbständigen kapillaren Flüssigkeitstransport ist es vorteilhaft, wenn der Mikrokanal eine lichte Breite im Bereich von 50 bis 500 µm aufweist.

Eine weitere vorteilhafte Ausgestaltung sieht vor, dass die Teilquerschnitte in dem Trägerkörper durch fotochemisches Maskenätzen gebildet sind.

Die Kapillarität lässt sich gemäß einer weiteren Erfindungsvariante auch dadurch erhöhen, dass der Mikrokanal einen vorzugsweise durch Hinterätzen gebildeten Hinterschnitt im Bereich seiner Längsränder aufweist.

Eine weitere Ausführung sieht vor, dass der Trägerkörper aus einem isotrop ätzbaren Material besteht, wobei sich speziell mit einem Flachformteil vorzugsweise aus Metall, insbesondere Edelstahl die gewünschten Eigenschaften wie günstige Handhabung, Festigkeit, Inertheit und Biokompatibilität realisieren lassen.

Vorteilhafterweise besitzt der aus Flachmaterial gebildete Trägerkörper eine Dicke von 100 bis 450 µm, vorzugsweise 150 bis 300 µm.

Vorteilhaft ist es auch, wenn die Aufbauschicht eine die Hydrophilie erhöhende Zusatzsubstanz oder Zusammensetzung aufweist, oder wenn die Benetzbarkeit einer Wand des Mikrokanals durch eine chemische Oberflächenbehandlung erhöht ist.

Eine weitere Verbesserung sieht vor, dass zumindest eine Teilstruktur des Trägerkörpers außerhalb des Mikrokanalbereichs, vorzugsweise das Stechorgan durch Ätzen oder Stanzen gebildet ist, so dass die verschiedenen Strukturierungen durch einheitliche Abläufe geschaffen werden.

Eine bevorzugte Einsatzmöglichkeit betrifft ein disposibles Probenentnahmeelement umfassend ein erfindungsgemäßes Mikrofluidiksystem.

Eine weitere bevorzugte Verwendung eines erfindungsgemäßen Mikrofluidiksystems liegt im Transport einer Probenflüssigkeit von einer Aufnahmezu einer Zielstelle, insbesondere zur Entnahme einer Körperflüssigkeit und zum Transport in einen Detektionsbereich.

In verfahrensmäßiger Hinsicht wird die eingangs genannte Aufgabe dadurch gelöst, dass durch eine auf einen Trägerkörper aufgebrachte Fotolackschicht, insbesondere einen Dickfilm-Fotolack ein Flüssigkeit transportierender Mikrokanal erhöht oder gebildet wird.

In vorteilhafter Ausgestaltung wird der Fotolack als Dickfilm auf den Trägerkörper aufgesprüht oder aufgerakelt oder durch Tauchbeschichtung aufgebracht.

Eine weitere vorteilhafte Maßnahme besteht darin, dass durch Maskenätzen einer ersten Fotolackschicht ein Mikrokanal in den Trägerkörper eingeätzt wird, und dass nach Entfernen der ersten Fotolackschicht eine zweite Fotolackschicht aufgebracht und zur Erhöhung des Mikrokanals fotostrukturiert wird.

Im Folgenden wird die Erfindung anhand der in der Zeichnung schematisch dargestellten Ausführungsbeispiele näher erläutert. Es zeigen
- Fig. 1: ein Probenentnahmeelement als Mikrofluidiksystem zum Transport einer Probenflüssigkeit in perspektivischer Darstellung;
- Fig. 2 bis 4: das System nach Fig. 1 mit unterschiedlicher Aufbauschicht eines Mikrokanals im Querschnitt;
- Fig. 5a bis f: aufeinander folgende Verfahrensschritte zur kanalerhöhenden Fotostrukturierung des Systems nach Fig. 1 im Querschnitt; und
- Fig. 6a bis k: aufeinander folgende Verfahrensschritte zur Kanalvertiefung in Fig. 5 entsprechender Darstellung.

Das in der Zeichnung dargestellte Mikrofluidiksystem ermöglicht als disposibles Probenentnahmeelement 10 die Entnahme und den Kapillartransport kleiner Körperflüssigkeitsmengen. Es umfasst hierzu einen flachen Trägerkörper 12, ein daran ausgebildetes Stechorgan 14 und einen kapillaren Mikrokanal 16, der durch eine Aufbauschicht 18 des Trägerkörpers 12 zumindest bereichsweise begrenzt sein kann.

Der Trägerkörper 12 besteht als streifenförmiges Flachformteil aus Stahl mit einer Dicke von etwa 150 bis 300 µm. Sein proximaler Endabschnitt bildet einen Haltebreich 20 für die Handhabung beim Stechvorgang, während das am distalen Ende einstückig angeformte Stechorgan 14 in der Haut eines Benutzers eine kleine Wunde erzeugt, um mikroskopische Volumina an Blut oder Gewebeflüssigkeit entnehmen zu können.

Der Mikrokanal 16 ist über seine Länge rillenförmig bzw. halboffen ausgebildet, so dass wie nachstehend beschrieben eine fotolithografische Herstellung möglich ist. An der Aufnahmestelle 22 im Bereich des Stechorgans (Lanzettenspitze 14) ist über den halboffenen Querschnitt eine effektive Flüssigkeitsaufnahme aus der Haut oder von der Hautoberfläche weg möglich, ohne dass Gewebeteile den Eintrittsquerschnitt wie bei herkömmlichen Hohlkanülen vollständig verschließen könnten.

Der Flüssigkeitstransport erfolgt über den Kapillarkanal 16 zu der im Abstand vom Stechorgan 14 liegenden Zielstelle 24, an der eine Analyse der Körperflüssigkeit stattfinden kann. Dies lässt sich beispielsweise durch reflexionsspektroskopische oder elektrochemische Nachweismethoden in an sich bekannter Weise realisieren.

Der Kanalquerschnitt kann über die Länge des Mikrokanals 16 konstant sein oder variieren. Bevorzugt liegt die Breite des Kanals im Bereich von 50 bis 500 µm, während das so genannte Aspektverhältnis zwischen Tiefe und Breite im Sinne einer verbesserten Kapillarität größer als 0,5, vorzugsweise größer als 0,8 ist. Hierbei ist zu berücksichtigen, dass beim isotropen Einätzen des Kanals 16 in den Trägerkörper 12 ein ungefähr halbkreisförmiger Querschnitt erhalten wird, bei dem das Aspektverhältnis lediglich 0,5 beträgt.

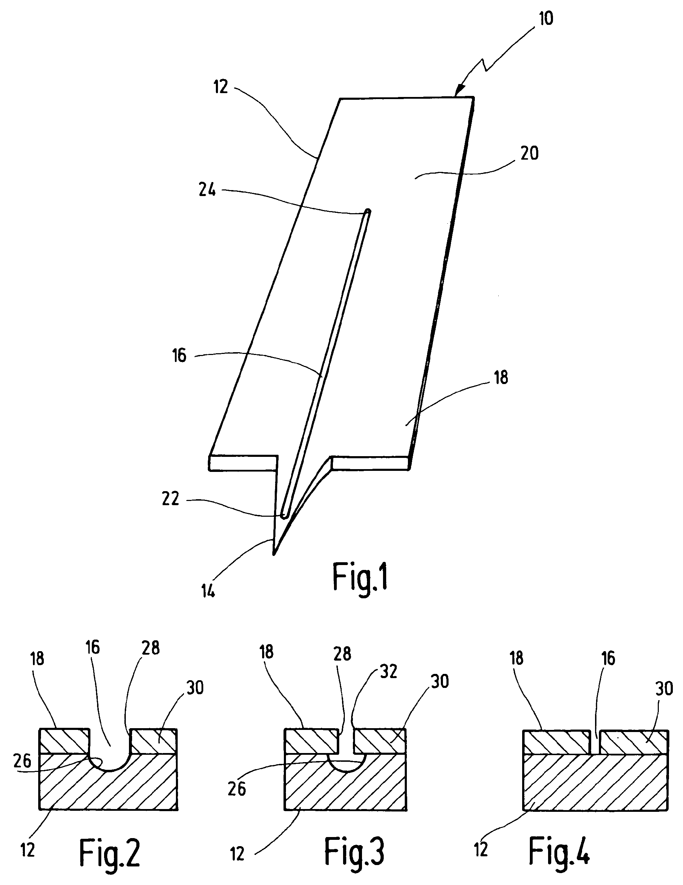

Wie in Fig. 2 dargestellt, kann der durch isotropes Ätzen gebildete halbkreisförmige untere Kanalbereich 26 in dem Trägerkörper bzw. Substrat 12 durch die Aufbauschicht 18 unter seitlicher Begrenzung eines oberen randoffenen Kanalbereichs 28 erhöht werden, so dass insgesamt ein höheres Aspektverhältnis und damit eine bessere Kapillarwirkung für den Flüssigkeitstransport erzielt wird. Die Aufbauschicht 18 sollte zu diesem Zweck eine Schichtdicke von mehr als 50 µm, vorzugsweise 200 bis 500 µm aufweisen.

Die Aufbauschicht 18 ist nicht als vorgefertigter Körper auf den Trägerkörper 12 auflaminiert, sondern aus einem zuvor formlosen Stoff als fest haftende Beschichtung aufgebracht. Hierfür ist ein flüssiger Beschichtungsstoff vorgesehen, speziell ein Fotolack 30 (engl. "Photoresist"). Besonders geeignet ist ein Dickfilm-Fotolack, beispielsweise auf Epoxybasis.

Bei der Ausführung nach Fig. 2 ist der Fotolack 30 nach dem Ätzen des unteren Bereichs 26 nachfolgend aufgebracht, so dass der komplementäre obere Kanalbereich 28 zusätzlich Flüssigkeit führen kann. Zu diesem Zweck kann es günstig sein, wenn die Hydrophilie der Schicht 18 durch geeignete Zusatzstoffe oder eine entsprechende Lackzusammensetzung erhöht wird. Möglich ist es auch, die Wasseraffinität der Kanalwandung durch eine chemische Oberflächenbehandlung nach der Strukturbildung zu verbessern.

In der Ausführung nach Fig. 3 wurde der für das Ätzen des unteren Bereichs 26 auf dem Trägerkörper 12 als Maske verwendete Photoresist 30 nicht entfernt, sondern für eine fluidische Zusatzfunktion beibehalten. Wie gezeigt, ist es neben der Erhöhung der Kanalwände auch möglich, die gegen Atmosphäre offene Fläche 32 zu reduzieren, wodurch die Kapillarität weiter erhöht wird. Grundsätzlich ist es auch denkbar, einen hinterschnittenen Randbereich des Kanals 16 als unterätzte Struktur des Trägerkörpers 12 durch geeignete Wahl der Ätzparameter herzustellen.

Fig. 4 zeigt ein Ausführungsbeispiel, bei dem die Aufbauschicht 18 den Mikrokanal 16 über seine gesamte Tiefe seitlich begrenzt, wobei auch hier durch eine entsprechende Schichtdicke des Fotolacks 30 ein hohes Aspektverhältnis erreichbar ist. Zusätzlich zu der Fotostrukturierung des Kanals 16 in der Schicht 18 kann durch vorgeschaltetes (isotropes) Ätzen der Trägerkörper 12 strukturiert werden, beispielsweise indem das Stechorgan 14 freigeätzt wird.

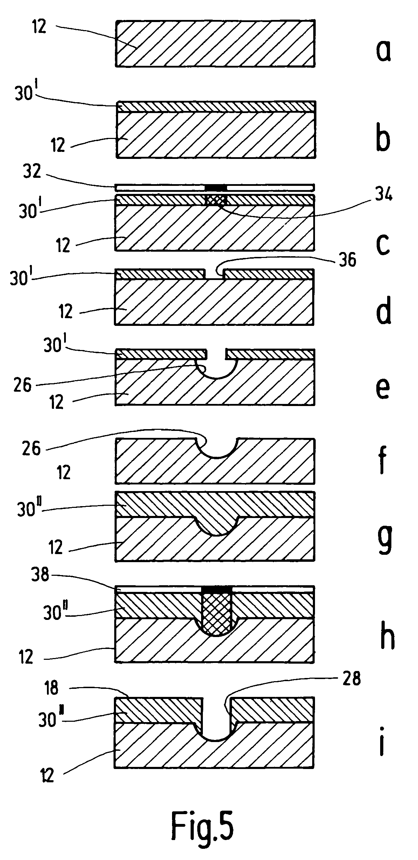

Fig. 5 veranschaulicht einen Verfahrensablauf zur Fotostrukturierung des Kanals 16 auf einer vorgängig geätzten Trägerstruktur. Zunächst wird der Trägerkörper 12 als Substrat mit einer ersten Fotolackschicht 30' versehen (Fig. 5a,b). Sodann erfolgt eine UV-Belichtung durch die Fotomaske 32 hindurch, wobei unter den lichtdurchlässigen Maskenbereichen der Fotolack 30' polymerisiert bzw. gehärtet wird, während die maskierten Bereiche 34 nach dem Belichten und Entwickeln freigespült werden (Fig. 5c,d). Über die so erzeugte Freisparung 36 in der Schicht 30' erfolgt anschließend eine Beaufschlagung des Trägerkörpers 12 mit einem Ätzmittel, wobei der Kanalbereich 26 isotrop freigeätzt wird. Nach dem Entfernen der Fotolackschicht 30' (Fig. 5f) wird durch eine weitere Fotostrukturierung einer zweiten Dickfilm-Schicht 30" mittels Maske 38 entsprechend dem bereits vorgeätzten Kanalverlauf eine Kanalerhöhung 28 gebildet (Fig. 5i). Der gehärtete Fotolack verbleibt als Aufbauschicht 18 permanent auf dem Substrat 12 und erfüllt damit eine fluidische Funktion für einen verbesserten Flüssigkeitstransport.

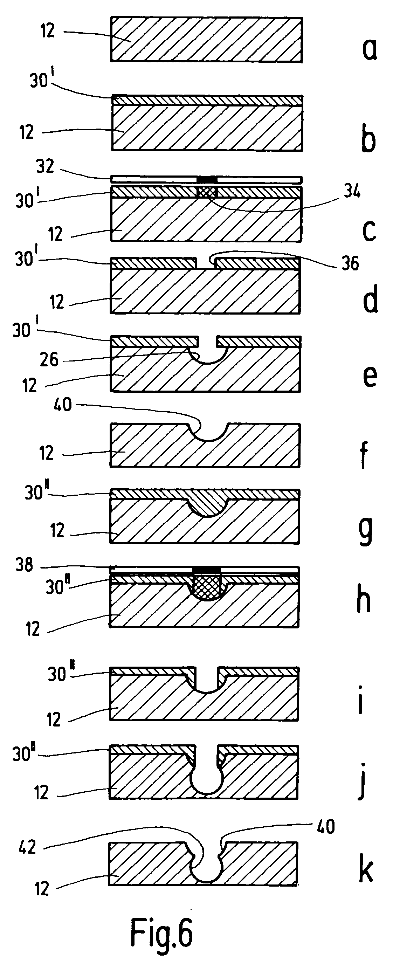

Bei dem in Fig. 6 dargestellten Verfahrensablauf wird durch mehrere sukzessive Ätzschritte das Aspektverhältnis des Kanals 16 erhöht. Durch eine erste Ätzung wird entsprechend der vorstehenden Beschreibung zu Fig. 5a bis f ein oberer Teilquerschnitt 40 des Kanals 16 in dem Trägerkörper 12 gebildet (Fig. 6a bis f). Sodann wird durch mindestens einmaliges Wiederholen dieser Schritte in einer zweiten oder weiteren Ätzung ein vertiefter Teilquerschnitt 42 erzeugt, so dass der Kanal 16 nahezu den gesamten Trägerkörper 12 durchsetzt, ohne sich jedoch isotrop in die Breite zu erstrecken (Fig. 6g bis k). Grundsätzlich ist es möglich, die Ätzungen parallel von beiden Seiten des Trägerkörpers 12 her gegeneinander zu führen, bis der Kanal 16 komplett durchgeätzt ist, wobei dann zumindest die Bodenseite beispielsweise durch Auflaminieren einer Folie verschlossen werden muss.

## Patentansprüche

1. Mikrofluidiksystem insbesondere zur Entnahme einer Körperflüssigkeit mit einem vorzugsweise mit einem Stechorgan (14) versehenen Trägerkörper (12) und einem darauf befindlichen halboffenen Mikrokanal (16) zum kapillaren Transport einer Flüssigkeit von einer Aufnahme- zu einer Zielstelle (22,24), **dadurch gekennzeichnet, dass** der Trägerkörper (12) mit einer den Mikrokanal (16) zumindest im oberen Bereich seitlich begrenzenden Aufbauschicht (18) für den Flüssigkeitstransport beschichtet ist.

2. Mikrofluidiksystem nach Anspruch 1, **dadurch gekennzeichnet, dass** der Mikrokanal (16) einen in den Trägerkörper (12) eingebrachten, vorzugsweise eingeätzten unteren Querschnittsbereich (26) und einen darüber liegenden, in der Aufbauschicht (18) ausgebildeten oberen Querschnittsbereich (28) aufweist.

3. Mikrofluidiksystem nach Anspruch 1 oder 2, **dadurch gekennzeichnet, dass** die Aufbauschicht (18) den Mikrokanal (16) über seine gesamte Tiefe seitlich begrenzt.

4. Mikrofluidiksystem nach einem der Ansprüche 1 bis 3, **dadurch gekennzeichnet, dass** die Aufbauschicht (18) aus einem Fotolack (30), vorzugsweise einem Dickfilm-Fotolack besteht.

5. Mikrofluidiksystem nach einem der Ansprüche 1 bis 4, **dadurch gekennzeichnet, dass** die Aufbauschicht (18) zur Bildung oder Erhöhung des Mikrokanals (16) fotostrukturiert ist.

6. Mikrofluidiksystem nach einem der Ansprüche 1 bis 5, **dadurch gekennzeichnet, dass** die Aufbauschicht (18) eine Schichtdicke von mehr als 50 µm, vorzugsweise 200 bis 500 µm aufweist.

7. Mikrofluidiksystem insbesondere zur Entnahme einer Körperflüssigkeit mit einem vorzugsweise mit einem Stechorgan (14) versehenen Trägerkörper (12) und einem darauf befindlichen halboffenen Mikrokanal (16) zum kapillaren Transport einer Flüssigkeit von einer Aufnahme- zu einer Zielstelle (22,24), **dadurch gekennzeichnet, dass** der Mikrokanal (16) mehrere durch sukzessive Ätzschritte von einer Oberfläche des Trägerkörpers (12) her in die Tiefe eingeätzte Teilquerschnitte (40,42) aufweist.

8. Mikrofluidiksystem nach einem der Ansprüche 1 bis 7, **dadurch gekennzeichnet, dass** das Aspektverhältnis aus Tiefe zu Breite des Mikrokanals (16) größer als 0,5, vorzugsweise größer als 0,8 ist.

9. Mikrofluidiksystem nach einem der Ansprüche 1 bis 8, **dadurch gekennzeichnet, dass** der Mikrokanal (16) eine lichte Breite im Bereich von 50 bis 500 µm aufweist.

10. Mikrofluidiksystem nach einem der Ansprüche 7 bis 9, **dadurch gekennzeichnet, dass** die Teilquerschnitte (40,42) durch fotochemisches Maskenätzen gebildet sind.

11. Mikrofluidiksystem nach einem der Ansprüche 1 bis 10, **dadurch gekennzeichnet, dass** der Mikrokanal (16) einen vorzugsweise durch Hinterätzen gebildeten Hinterschnitt im Bereich seiner Längsränder aufweist.

12. Mikrofluidiksystem insbesondere zur Entnahme einer Körperflüssigkeit mit einem vorzugsweise mit einem Stechorgan (14) versehenen Trägerkörper (12) und einem darauf befindlichen halboffenen Mikrokanal (16) zum kapillaren Transport einer Flüssigkeit von einer Aufnahme- zu einer Zielstelle (22,24), **dadurch gekennzeichnet, dass** der Mikrokanal (16) einen vorzugsweise durch Hinterätzen gebildeten Hinterschnitt im Bereich seiner Längsränder aufweist.

13. Mikrofluidiksystem nach einem der Ansprüche 1 bis 12, **dadurch gekennzeichnet, dass** der Trägerkörper (12) aus einem isotrop ätzbaren Material besteht.

14. Mikrofluidiksystem nach einem der Ansprüche 1 bis 13, **dadurch gekennzeichnet, dass** der Trägerkörper (12) als Flachformteil vorzugsweise aus Metall, insbesondere Edelstahl besteht.

15. Mikrofluidiksystem nach einem der Ansprüche 1 bis 14, **dadurch gekennzeichnet, dass** der aus Flachmaterial gebildete Trägerkörper (12) eine Dicke von 100 bis 450 µm, vorzugsweise 150 bis 300 µm aufweist.

16. Mikrofluidiksystem nach einem der Ansprüche 1 bis 15, **dadurch gekennzeichnet, dass** die Aufbauschicht (18) eine die Hydrophilie erhöhende Zusatzsubstanz oder Zusammensetzung aufweist.

17. Mikrofluidiksystem nach einem der Ansprüche 1 bis 16, **dadurch gekennzeichnet, dass** die Benetzbarkeit einer Wand des Mikrokanals (16) durch eine chemische Oberflächenbehandlung erhöht ist.

18. Mikrofluidiksystem nach einem der Ansprüche 1 bis 17, **dadurch gekennzeichnet, dass** zumindest eine Teilstruktur des Trägerkörpers (12) außerhalb des Mikrokanalbereichs, vorzugsweise das Stechorgan (14) durch Ätzen oder Stanzen gebildet ist.

19. Disposibles Probenentnahmeelement umfassend ein Mikrofluidiksystem (10) nach einem der vorhergehenden Ansprüche.

20. Verwendung eines Mikrofluidiksystems (10) nach einem der vorhergehenden Ansprüche zum Transport einer Probenflüssigkeit von einer Aufnahme- zu einer Zielstelle (22,24), insbesondere zur Entnahme einer Körperflüssigkeit und zum Transport in einen Detektionsbereich.

21. Verfahren zur Herstellung eines Mikrofluidiksystems bei welchem durch eine auf einen Trägerkörper (12) aufgebrachte Fotolackschicht, insbesondere einen Dickfilm-Fotolack (30) ein Flüssigkeit transportierender Mikrokanal (16) erhöht oder gebildet wird.

22. Verfahren nach Anspruch 21, **dadurch gekennzeichnet, dass** der Fotolack (30) als Dickfilm auf den Trägerkörper (12) aufgesprüht oder aufgerakelt oder durch Tauchbeschichtung aufgebracht wird.

23. Verfahren nach Anspruch 21 oder 22, **dadurch gekennzeichnet, dass** durch Maskenätzen einer ersten Fotolackschicht (30') ein Mikrokanal (16) in den Trägerkörper (12) eingeätzt wird, und dass nach Entfernen der ersten Fotolackschicht eine zweite Fotolackschicht (30") aufgebracht und zur Erhöhung des Mikrokanals (16) fotostrukturiert wird.
